(19) Europäisches Patentamt
European Patent Office
Office européen des brevets

(11) Veröffentlichungsnummer: **0 346 280 B1**

# (12) EUROPÄISCHE PATENTSCHRIFT

(45) Veröffentlichungstag der Patentschrift: **09.11.94**

(51) Int. Cl.$^5$: **C09B 3/04**, D06P 1/22

(21) Anmeldenummer: **89810404.7**

(22) Anmeldetag: **30.05.89**

Die Akte enthält technische Angaben, die nach dem Eingang der Anmeldung eingereicht wurden und die nicht in dieser Patentschrift enthalten sind.

## (54) Dicyanobenzanthronverbindungen.

(30) Priorität: **06.06.88 CH 2136/88**

(43) Veröffentlichungstag der Anmeldung:
**13.12.89 Patentblatt 89/50**

(45) Bekanntmachung des Hinweises auf die Patenterteilung:
**09.11.94 Patentblatt 94/45**

(84) Benannte Vertragsstaaten:
**CH DE FR GB IT LI**

(56) Entgegenhaltungen:
**EP-A- 0 238 443**

(73) Patentinhaber: **CIBA-GEIGY AG**
**Klybeckstrasse 141**
**CH-4002 Basel (CH)**

(72) Erfinder: **Adam, Jean-Marie, Dr.**
**Rue de Village Neuf 60 D**
**F-68300 Rosenau (FR)**

EP 0 346 280 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Dicyanobenzanthronverbindungen, Verfahren zu deren Herstellung sowie deren Verwendung als Farbstoffe zum Färben und Bedrucken von halbsynthetischem oder synthetischem hydrophobem Material.

Die neuen Dicyanobenzanthronverbindungen entsprechen der Formel

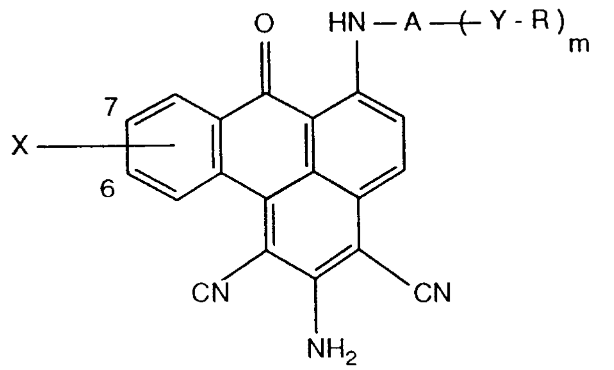

(1),

worin

X Wasserstoff, Halogen, Nitro oder $NH_2$ ist,

R Wasserstoff oder einen Rest $-COR^1$ bedeutet, worin $R^1$ eine gegebenenfalls substituierte Alkyl- oder Arylgruppe darstellt,

Y O oder S ist,

m 1, 2 oder 3 ist und

A einen aliphatischen, aromatischen oder araliphatischen Rest bedeutet, wobei falls R Wasserstoff, Y O, X Wasserstoff oder ein in der Position 6 oder 7 gebundenes Halogen und A ein aliphatischer Rest sind, darf A nur $C_2$- $C_5$-Alkylen bedeuten.

Bedeutet X Halogen, so ist darunter z.B. Brom oder vor allem Chlor zu verstehen. Das Halogen kann sich in 6- oder 7-Position befinden.

Vorzugsweise bedeutet X jedoch Wasserstoff.

Y bedeutet -S- oder vorzugsweise -O-.

Farbstoffe der Formel (1), bei denen R einen Rest $-COR^1$ bedeutet, stellen eine bevorzugte Ausführungsform der vorliegenden Erfindung dar.

Bedeutet $R^1$ einen Alkylrest, so handelt es sich um einen gegebenenfalls substituierten unverzweigten oder verzweigten Alkylrest oder um einen Cycloalkylrest. Der Cycloalkylrest weist vorzugsweise 5 bis 8 C-Atome, der offenkettige Alkylrest vorzugsweise 1 bis 8 C-Atome auf.

Als unverzweigte oder verzweigte offenkettige Alkylreste kommen z.B. in Frage: Methyl, Ethyl, n- und iso-Propyl, n-, sec.- oder tert.Butyl, n-und iso-Pentyl, n- und iso-Hexyl oder 2-Ethylhexyl.

Diese Alkylreste können ein- oder mehrmals substituiert sein, beispielsweise durch $C_1-C_4$-Alkoxy, Phenyl oder Phenoxy, wobei die Phenylgruppe in den beiden letztgenannten Resten z.B. durch $C_1$-$C_4$-Alkyl, $C_1$-$C_4$-Alkoxy, oder Phenoxy substituiert sein kann. Geeignete Reste dieser Art sind z.B.: Ethoxymethyl, Benzyl, 1-Phenylethyl, 2-Phenylethyl, 1-Methyl-2-phenylethyl, 1-iso-Butyl-3-phenylpropyl, 1,5-Diphenylpentyl-3 oder 1-Methyl-2-phenoxyethyl.

Bedeutet $R^1$ einen gegebenenfalls substituierten $C_5$-$C_8$-Cycloalkylrest, so handelt es sich vor allem um den Cyclopentyl- und Cyclohexylrest; als Substituenten kommen vor allem $C_1$-$C_4$-Alkylgruppen, insbesondere die $CH_3$-Gruppe, in Frage.

Bedeutet $R^1$ einen Arylrest, so handelt es sich vor allem um einen Naphthylrest und insbesondere um einen Phenylrest, wobei diese Reste substituiert sein können, z.B. durch $C_1$-$C_4$-Alkyl, $C_1$-$C_4$-Alkoxy, Halogen, wie Fluor, Chlor oder Brom, Nitro, $C_1$-$C_4$-Alkylcarbonylamino oder $C_1$-$C_4$-Alkoxycarbonyl.

In bevorzugten Farbstoffen der Formel (1) bedeutet

$R^1$ $C_1$-$C_6$-Alkyl, welches unsubstituiert ist oder durch Phenyl oder Phenoxy substituiert ist, wobei die Phenylgruppe in den beiden letztgenannten Resten durch $C_1$-$C_4$-Alkyl, $C_1$-$C_4$-Alkoxy oder Phenoxy substituiert sein kann, oder Phenyl, welches unsubstituiert ist oder durch $C_1$-$C_4$-Alkyl substituiert ist.

Besonders bevorzugt sind Farbstoffe der Formel (1) bei denen $R^1$ $C_1$-$C_3$-Alkyl oder Phenyl bedeutet.

Bevorzugte Farbstoffe der Formel (1) enthalten 1 oder 2, vor allem 1 Rest -Y-R.

Das Brückenglied A ist ein aliphatischer, aromatischer oder araliphatischer Rest, an welchen 1 bis 3 Gruppen -Y-R gebunden sind.

Geeignete aliphatische Reste sind beispielsweise geradkettiges oder verzweigtes $C_2$-$C_8$-Alkylen oder cyclisches $C_5$-$C_8$-Alkylen, beispielsweise Ethylen, 1,3- oder 1,2-Propylen, 1,4-, 2,3- oder 2,4-Butylen, 2-Methyl-1,3-propylen, 2-Methyl-2,4-pentylen, 2,2-Dimethyl-1,3-propylen, 1,6- oder 2,5-Hexylen, 1-Methyl-2,6-heptylen, 1,3- oder 1,4-Cyclohexylen. Geeignete aromatische Reste sind z.B. 1,3- oder 1,4-Phenylen, 1,4- oder 1,5-Naphthylen.

Als araliphatische Reste kommen beispielsweise in Frage: o-, m- und p-Phenylenmethylen, o-, m- und p-Phenylen-ethylen-2, Phenylethylen-1,2, 1-Phenylpropylen-1,3, 2-Phenylpropylen-1,3, 1-Phenylpropylen-1,2 und 1-Phenylpropylen-2,3, wobei die Reste -Y-R am aliphatischen und/oder aromatischen Teil gebunden sein können.

Vorzugsweise stellt A einen $C_2$-$C_5$-Alkylenrest dar oder einen Phenylen-$C_2$-$C_5$-alkylenrest, besonders bevorzugt einen Propylen-, Butylen-, Phenylenethylen- oder Phenylenpropylenrest.

Wegen ihrer guten färberischen Eigenschaften, insbesondere wegen des guten Ausziehvermögens aus wässrigem Färbebad, sind Farbstoffe der Formel

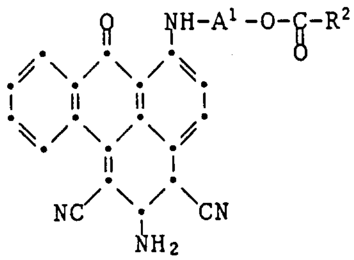

(2)

worin
$A^1$ einen $C_2$-$C_5$-Alkylenrest oder einen Phenyl-$C_2$-$C_5$-Alkylenrest und
$R^2$ $C_1$-$C_3$-Alkyl oder Phenyl
bedeutet, besonders bevorzugt.

Die neuen Dicyanobenzanthronverbindungen der Formel (1) erhält man z.B., indem man ein substituiertes Aminoanthrachinon der Formel

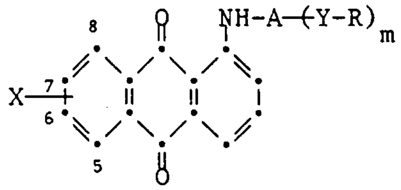

(3)

worin X, Y, A, m und R die unter der Formel (1) angegebene Bedeutung aufweisen, in einem inerten Lösungsmittel in Gegenwart von Titantetrachlorid und einem tert.Amin mit Malodinitril umsetzt.

Als inerte Lösungsmittel kommen z.B. aliphatische Kohlenwasserstoffe, wie n-Pentan, n-Hexan oder n-Heptan, chlorierte Kohlenwasserstoffe, wie Methylenchlorid, Chloroform oder Tetrachlormethan, Ether, wie z.B. Diethylether, oder aromatische Verbindungen, wie z.B. Nitrobenzol oder Halogenbenzole in Betracht. Vorzugsweise verwendet man einen chlorierten Kohlenwasserstoff, vor allem Methylenchlorid.
Als tert.Amin werden z.B. aliphatische Amine, wie Triethylamin oder vor allem aromatische Amine, wie z.B. Picolin oder Pyridin verwendet.

Pro Mol Anthrachinonverbindung der Formel (3) setzt man mindestens 2 Mol, vorzugsweise jedoch 2,2 bis 3 Mol Malodinitril ein. Ein grösserer Ueberschuss ist im allgemeinen nicht schädlich, jedoch unzweckmässig.

Die Menge an Titantetrachlorid beträgt vorzugsweise 2 bis 10 Mol, insbesondere 3 bis 6 Mol pro Mol Anthrachinonverbindung der Formel (3).

Das tertiäre Amin wird mindestens in der Menge eingesetzt, die erforderlich ist, um die entstehende Säure zu binden. Im allgemeinen verwendet man jedoch einen Ueberschuss, vorzugsweise 2 bis 6 Mol pro Mol Titantetrachlorid.

Die Reaktionstemperatur liegt im allgemeinen etwa zwischen -10° und +60°C, vorzugsweise zwischen 0° und 25°C.

Nach Beendigung der Reaktion wird der Farbstoff auf an sich bekannte Weise isoliert, z.B. indem man die flüchtigen Anteile der Reaktionsmischung abdestilliert, den Rückstand mit verdünnter wässriger Säure behandelt, abfiltriert und gegebenenfalls umkristallisiert.

Farbstoffe der Formel (1), bei denen R einen Rest

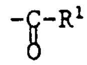

darstellt, können auch erhalten werden, indem man zunächst eine Verbindung der Formel (3), worin R Wasserstoff ist, wie unter der Formel (3) beschrieben zur Dicyanobenzanthronverbindung umsetzt und diese anschliessend auf übliche Weise acyliert.

Die erfindungsgemässen Verbindungen der Formel (1) können als Farbstoffe zum Färben und Bedrukken von halbsynthetischen und, insbesondere, synthetischen hydrophoben Fasermaterialien, vor allem Textilmaterialien, verwendet werden. Textilmaterialien aus Mischgeweben, die derartige halbsynthetische bzw. synthetische hydrophobe Textilmaterialien enthalten, können ebenfalls mit Hilfe der erfindungsgemässen Verbindungen gefärbt oder bedruckt werden.

Als halbsynthetische Textilmaterialien kommen vor allem Cellulose-$2\frac{1}{2}$ -Acetat und Cellulosetriacetat in Frage.

Synthetische hydrophobe Textilmaterialien bestehen vor allem aus linearen, aromatischen Polyestern, beispielsweise solchen aus Terephthalsäure und Glykolen, besonders Ethylenglykol oder Kondensationsprodukten aus Terephthalsäure und 1,4-Bis-(hydroxymethyl)-hexahydrobenzol; aus Polycarbonaten, z.B. solchen aus $\alpha,\alpha$-Dimethyl-4,4'-dihydroxy-diphenylmethan und Phosgen, aus Fasern auf Polyvinylchlorid- sowie Polyamid-Basis.

Die Applikation der erfindungsgemässen Verbindungen auf die Textilmaterialien erfolgt nach bekannten Färbeverfahren. Beispielsweise färbt man Polyesterfasermaterialien im Ausziehverfahren aus wässriger Dispersion in Gegenwart von üblichen anionischen oder nichtionischen Dispergiermitteln und gegebenenfalls üblichen Quellmitteln (Carrier) bei Temperaturen zwischen 80 und 140°C. Cellulose-$2\frac{1}{2}$ -acetat färbt man vorzugsweise zwischen ungefähr 65 bis 85°C und Cellulosetriacetat bei Temperaturen bis zu 115°C.

Die neuen Farbstoffe färben im Färbebad gleichzeitig anwesende Wolle und Baumwolle nicht oder nur wenig an (sehr gute Reserve), so dass sie auch gut zum Färben des Polyesteranteils in Polyester/Wolle- und Polyester/Cellulosefaser-Mischungen verwendet werden können.

Aus der EP-A-238.443 sind bereits Dicyanobenzanthronverbindungen bekannt. Diese unterscheiden sich von den erfindungsgemässen Verbindungen der Formel (1) durch die substituierte Aminogruppe. In der EP-A-238.443 sind keine Verbindungen mit einem Rest -Y-R gemäss vorliegender Erfindung offenbart.

Gegenüber den bekannten Farbstoffen zeichnen sich die erfindungsgemässen Farbstoffe, enthaltend mindestens eine Gruppe -Y-R, durch erheblich verbessertes Ausziehvermögen auf Polyestermaterial aus. Während die bekannten Farbstoffe für das wässrige Ausziehverfahren, vor allem zum Färben in tiefen Nuancen, nur bedingt geeignet sind, ergeben die erfindungsgemässen Farbstoffe auch im Ausziehverfahren sehr gute Fixierausbeuten.

Die erfindungsgemässen Farbstoffe der Formel (1) eignen sich auch zum Färben nach dem Thermosol-Verfahren und für den Textildruck.

Das genannte Textilmaterial kann in den verschiedensten Verarbeitungsformen vorliegen, wie z.B. als Faser, Faden oder Vlies, als Gewebe oder Gewirke.

Es ist vorteilhaft, die erfindungsgemässen Farbstoffe vor ihrer Verwendung in ein Farbstoffpräparat überzuführen. Hierzu wird der Farbstoff vermahlen, so dass seine Teilchengrösse im Mittel zwischen 0,01 und 10 Mikrometer beträgt. Das Vermahlen kann in Gegenwart von Dispergiermitteln erfolgen. Beispielsweise wird der getrocknete Farbstoff mit einem Dispergiermittel gemahlen oder in Pastenform mit einem Dispergiermittel geknetet und hierauf im Vakuum oder durch Zerstäuben getrocknet. Mit den so erhaltenen Präparaten kann man nach Zugabe von Wasser färben und bedrucken.

Beim Bedrucken wird man die üblichen Verdickungsmittel verwenden, z.B. modifizierte oder nichtmodifizierte natürliche Produkte, beispielsweise Alginate, Britishgummi, Gummi arabicum, Kristallgummi, Johannisbrotkernmehl, Tragant, Carboxymethylcellulose, Hydroxyethylcellulose, Stärke oder synthetische Produkte, beispielsweise Polyacrylamide, Polyacrylsäure oder deren Copolymere oder Polyvinylalkohole.

Die erfindungsgemässen Farbstoffe sind praktisch unempfindlich gegen Carrier und verleihen den genannten Materialien, vor allem dem Polyestermaterial egale blaustichig rot bis violette Farbtöne von sehr guten Gebrauchs-Echtheiten, wie vor allem guter Lichtechtheit, guter Sublimierechtheit, Thermofixier-, Plissier-, Chlor- und Nassechtheit wie Wasser-, Schweiss- und Waschechtheit; die Ausfärbungen sind ferner gekennzeichnet durch eine gute pH-Stabilität und sehr gute Reibechtheit. Es werden ausserdem sehr farbstarke Färbungen erzielt, die kein "catalytic fading" zeigen.

Die erfindungsgemässen Farbstoffe können auch gut verwendet werden zur Herstellung von Mischnuancen zusammen mit anderen Farbstoffen. Selbstverständlich können auch Mischungen der erfindungsgemässen Farbstoffe untereinander verwendet werden.

Die neuen Farbstoffe eignen sich auch zum Färben und Pigmentieren von hochmolekularen organischen Materialien, z.B. von Celluloseäthern und -estern, wie Aethylcellulose, Nitrocellulose, Celluloseacetat, Cellulosebutyrat, natürlichen Harzen oder Kunstharzen, wie Polymerisationsharzen oder Kondensationsharzen, z.B. Aminoplasten, insbesondere Harnstoff- und Melamin-Formaldehydharzen, Alkydharzen, Phenoplasten, Polycarbonaten, Polyolefinen, wie Polystyrol, Polyvinylchlorid, Polyethylen, Polypropylen, Polyacrylnitril, Polyacrylsäureester, Polyamiden, Polyurethanen oder Polyestern, Gummi, Casein, Silikon und Silikonharzen, einzeln oder in Mischungen.

Dabei spielt es keine Rolle, ob die erwähnten hochmolekularen Verbindungen als plastische Massen, Schmelzen oder in Form von Spinnlösungen, Lacken, Anstrichstoffen oder Druckfarben vorliegen. Je nach Verwendungszweck erweist es sich als vorteilhaft, die neuen Farbstoffe als Toner oder in Form von Präparaten zu verwenden.

Die Farbstoffe können in der Form, wie sie in der Synthese anfallen, eingesetzt werden. In leicht gemahlener Form liefern sie opake Ausfärbungen. Man kann sie aber auch einer intensiveren Mahlung unterziehen und erhält dann transparente Ausfärbungen, wie beispielsweise farbstarke Metalleffektlackierung.

Thermoplastische Kunststoffe, die mit den neuen Farbstoffen der Formel (1) in der Schmelze gefärbt werden können, sind Polystyrol und deren Mischpolymerisate, Polycarbonate, Polyamide, insbesondere aber lineare Polyester.

Als lineare Polyester seien insbesondere jene erwähnt, die durch Polykondensation von Terephthalsäure oder deren Estern mit Glykolen der Formel $HO-(CH_2)_n-OH$, worin n die Zahl 2-10 bedeutet, oder mit 1,4-Di-(hydroxymethyl)-cyclohexan oder durch Polykondensation von Glykolethern von Hydroxybenzoesäuren, beispielsweise p-(β-Hydroxyethoxy)-benzoesäure erhalten werden. Der Begriff lineare Polyester umfasst auch Copolyester, die durch teilweisen Ersatz der Terephthalsäure durch eine andere Dicarbonsäure oder eine Hydroxycarbonsäure und/oder durch teilweisen Ersatz des Glykols durch ein anderes Diol erhalten werden.

Von besonderem Interesse sind jedoch die Polyethylen-terephthalate.

Die zu färbenden linearen Polyester werden zweckmässig in Form von Pulver, Schnitzeln oder Granulaten mit dem Farbstoff innig vermischt. Dies kann beispielsweise durch Panieren der Polyesterteilchen mit dem fein verteilten trockenen Farbstoffpulver oder durch Behandeln der Polyesterteilchen mit einer Lösung oder Suspension des Farbstoffes in einem organischen Lösungsmittel und nachherige Entfernung des Lösungsmittels geschehen.

Schliesslich kann die zu färbende Substanz auch direkt dem geschmolzenen Polyester oder auch vor oder während der Polykondensation des Polyethylenterephthalates zugegeben werden.

Das Verhältnis von Farbstoff zu Polyester kann, je nach der gewünschten Farbstärke, innerhalb weiter Grenzen schwanken. Im allgemeinen empfiehlt sich die Verwendung von 0,01-2 Teilen Farbstoff auf 100 Teile Polyester.

Die so behandelten Polyesterteilchen werden nach bekannten Verfahren im Extruder geschmolzen und zu Gegenständen, insbesondere Folien oder Fasern, ausgepresst oder zu Platten gegossen.

Man erhält gleichmässig und intensiv blaustichig rot bis violett gefärbte Gegenstände von hoher Licht- oder Migrationsechtheit. Die verfahrensgemäss erhältlichen gefärbten Fasern zeichnen sich ausserdem durch hervorragende Nass- und Trockenreinigungsechtheiten aus.

Ein besonderer Vorteil der erfindungsgemäss zu verwendenden Farbstoffe besteht darin, dass sie sich in der Polyesterschmelze lösen und hohe Temperaturen bis 300 °C aushalten, ohne sich zu zersetzen, so dass bedeutend klarere Färbungen erhalten werden als bei Verwendung unlöslicher Pigmente.

Die vorstehend genannten Verwendungen der erfindungsgemässen Dicyanobenzanthronverbindungen der Formel (1) stellen ebenso Gegenstände der vorliegenden Erfindung dar wie ein Verfahren zum Färben oder Bedrucken von halbsynthetischem oder synthetischem Fasermaterial, insbesondere Textilmaterial, das darin besteht, eine oder mehrere Verbindungen der Formel (1) auf das genannte Material aufzubringen oder es diesem einzuverleiben. Das genannte hydrophobe Fasermaterial ist vorzugsweise textiles Polyestermaterial. Weitere Substrate, die durch das erfindungsgemässe Verfahren behandelt werden können sowie bevorzugte Verfahrensbedingungen sind vorstehend bei der näheren Erläuterung der Verwendung der erfindungsgemässen Verbindungen zu finden.

Ein weiterer Gegenstand der Erfindung ist das durch das genannte Verfahren gefärbte bzw. bedruckte hydrophobe Fasermaterial, vorzugsweise Polyester-Textilmaterial.

Die folgenden Beispiele veranschaulichen die Erfindung weiter. Teile und Prozente beziehen sich, sofern nicht anderes angegeben ist, auf das Gewicht. Die Temperaturen sind in Grad Celsius angegeben.

Beispiel 1: Zu einem Gemisch, bestehend aus 11,8 g der Verbindung der Formel

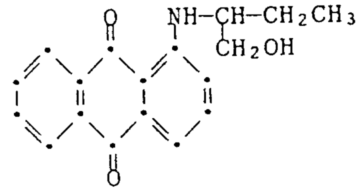

7 g Malodinitril und 250 ml Methylenchlorid lässt man bei einer Temperatur von 0-5° unter intensivem Rühren innerhalb von 30 Minuten 23 ml Titantetrachlorid zutropfen und anschliessend bei derselben Temperatur innerhalb von 30 Minuten 70 ml Pyridin. Man rührt das Gemisch anschliessend noch während 2 Stunden, wobei man die Temperatur auf 20° ansteigen lässt. Man entfernt nun die flüchtigen Anteile in einem Rotationsverdampfer im Vakuum, worauf man den Rückstand mit 200 ml 2n-HCl behandelt, abfiltriert und mit 2n-HCl nachwäscht. Man wäscht hierauf das Nutschgut mit Wasser bis zum neutralen Ablauf und anschliessend mit Methanol zur Entfernung brauner Nebenprodukte. Nach Zwischentrocknung wird das Produkt aus 10 Teilen Ethylcellosolve umkristallisiert. Man erhält den Farbstoff als dunkelviolettes Pulver.

Der Farbstoff entspricht der Formel

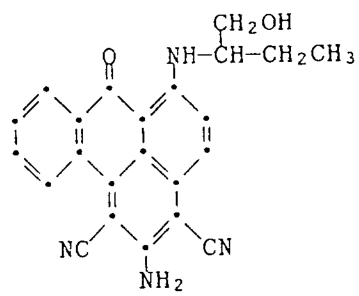

welche sich in Uebereinstimmung befindet mit der Elementaranalyse, dem Massen-, dem Protonen-Kernresonanz-Spektrum.

Als Dispersionsfarbstoff nach den üblichen Ausziehverfahren auf Polyester appliziert erhält man brillante blaustichigrote Färbungen mit guten Echtheitseigenschaften, insbesondere mit guten Sublimations- und Lichtechtheiten bei hohem Ausziehgrad.

Beispiele 2-23: Verwendet man, bei ansonst gleichem Vorgehen, entsprechende durch OH-Gruppen substituierte 1-Alkylamino-, 1-Aralkylamino- bzw. Arylamino-Anthrachinone, so erhält man die in der folgenden Tabelle aufgeführten Farbstoffe, welche ähnliche Eigenschaften aufweisen und Polyester in blaustichig roten Nuancen färben.

Tabelle 1

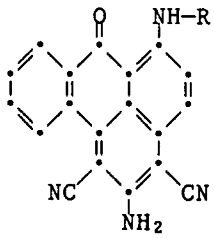

| Beispiel | R |
|---|---|
| 2 | 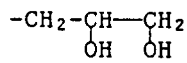 |
| 3 | 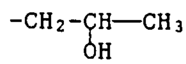 |
| 4 | 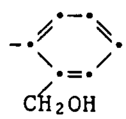 |
| 5 | 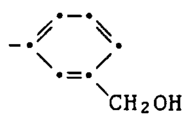 |
| 6 | 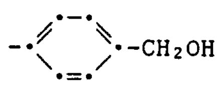 |
| 7 | 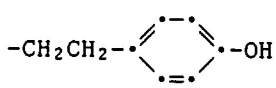 |
| 8 | 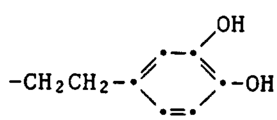 |
| 9 | $-CH_2CH_2OH$ |
| 10 | 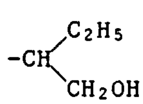 |
| 11 | $-CH_2CH_2CH_2CH_2OH$ |
| 12 | 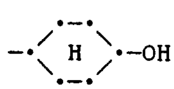 |

Tabelle 1 (Fortsetzung)

| Beispiel | R |
|---|---|
| 13 | 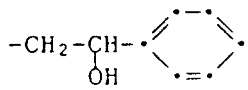 |
| 14 | 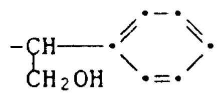 |
| 16 | 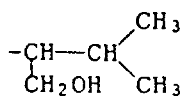 |
| 17 | 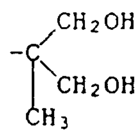 |
| 18 | 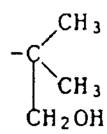 |
| 19 | 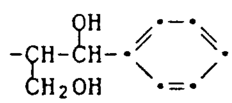 |
| 20 | 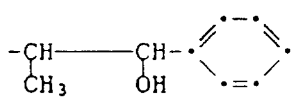 |
| 21 | 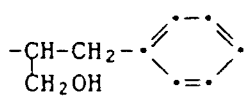 |
| 22 | 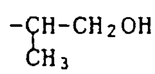 |
| 23 | $-CH_2CH_2CH_2-OH$ |

Beispiel 24: 3,6 g des Farbstoffes gemäss Beispiel 1 werden in 50 ml Pyridin gelöst und bei 20 °C mit 1,8 ml Essigsäureanhydrid versetzt. Man rührt noch 15 Std. bei 20 °C und verdünnt mit 50 ml Wasser.

Der ausgefallene Farbstoff der Formel

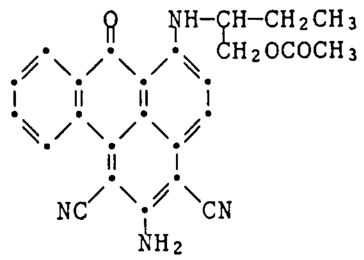

wird abgesaugt mit Wasser gewaschen und getrocknet.

Als Dispersionsfarbstoff nach den üblichen Verfahren auf Polyester appliziert erhält man brillante blaustichig-rote Färbungen mit guten Echtheitseigenschaften, insbesondere mit guten Sublimations- und Lichtechtheiten und sehr gutem Ausziehgrad.

Ersetzt man im obigen Beispiel das Essigsäureanhydrid durch Propionsäure- oder Benzoesäureanhydrid so erhält man ähnliche Farbstoffe mit gleich guten Eigenschaften.

Beispiele 25-90: Verwendet man, bei ansonst gleichem Vorgehen, entsprechende durch Acyloxy-Gruppen substituierte 1-Alkylamino- bzw. 1-Aryl-oder 1-Aralkylaminoanthrachinone, so erhält man die in der folgenden Tabelle aufgeführten Farbstoffe, welche ähnliche Eigenschaften aufweisen und Polyester in der angegebenen Nuance färben.

Tabelle 2

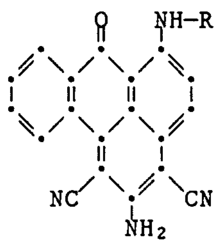

| Beispiel | R (Ac = $\overset{O}{\overset{\|}{C}}$-$CH_3$) | Nuance auf Polyester |
|---|---|---|
| 25 | 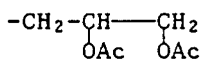 | blaustichig-rot |
| 26 | 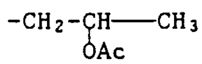 | blaustichig-rot |
| 27 | 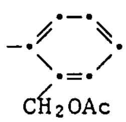 | rot-violett |
| 28 | 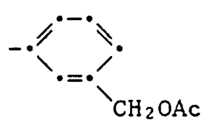 | rot-violett |
| 29 | 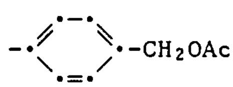 | rot-violett |
| 30 | 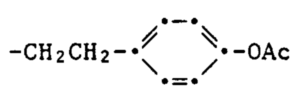 | blaustichig-rot |
| 31 | 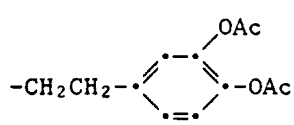 | blaustichig-rot |
| 32 | $-CH_2CH_2OAc$ | blaustichig-rot |
| 33 | 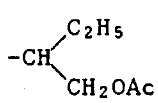 | blaustichig-rot |
| 34 | $-CH_2CH_2CH_2CH_2OAc$ | blaustichig-rot |
| 35 | 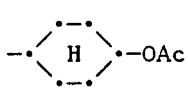 | blaustichig-rot |

Tabelle 2 (Fortsetzung)

| Beispiel | R (Ac = 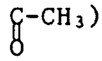) | Nuance auf Polyester |
|---|---|---|
| 36 | 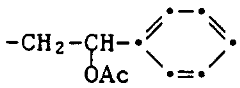 | blaustichig-rot |
| 37 | 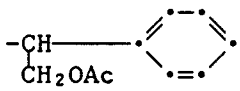 | blaustichig-rot |
| 38 | 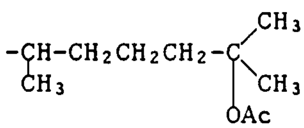 | blaustichig-rot |
| 39 | 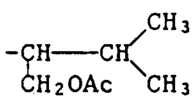 | blaustichig-rot |
| 40 | 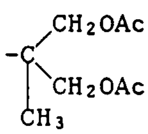 | blaustichig-rot |
| 41 | 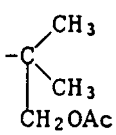 | blaustichig-rot |
| 42 | 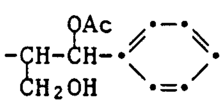 | blaustichig-rot |
| 43 | 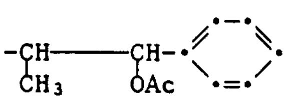 | blaustichig-rot |
| 44 | 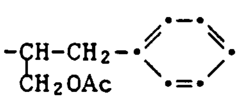 | blaustichig-rot |
| 45 | 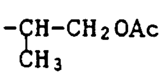 | blaustichig-rot |
| 46 | $-CH_2CH_2CH_2-OAc$ | blaustichig-rot |

Tabelle 3

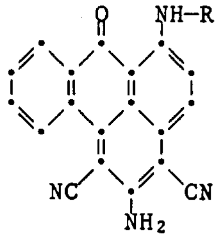

| Beispiel | R (Pr = 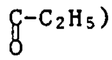) | Nuance auf Polyester |
|---|---|---|
| 47 | 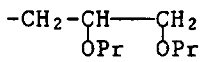 | blaustichig-rot |
| 48 | 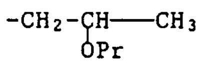 | blaustichig-rot |
| 49 | 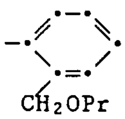 | rot-violett |
| 50 | 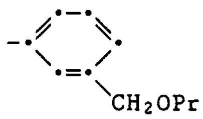 | rot-violett |
| 51 | 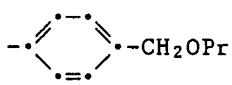 | rot-violett |
| 52 | 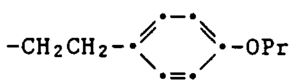 | blaustichig-rot |
| 53 | 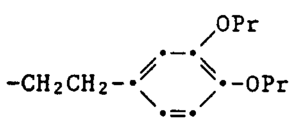 | blaustichig-rot |
| 54 | $-CH_2CH_2OPr$ | blaustichig-rot |
| 55 | 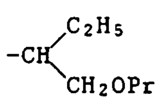 | blaustichig-rot |
| 56 | $-CH_2CH_2CH_2CH_2OPr$ | blaustichig-rot |
| 57 | 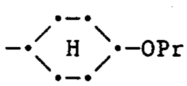 | blaustichig-rot |

Tabelle 3 (Fortsetzung)

| Beispiel | R (Pr = 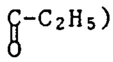) | Nuance auf Polyester |
|---|---|---|
| 58 | 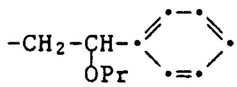 | blaustichig-rot |
| 59 | 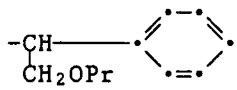 | blaustichig-rot |
| 60 | 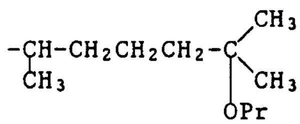 | blaustichig-rot |
| 61 | 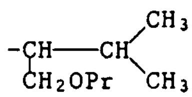 | blaustichig-rot |
| 62 | 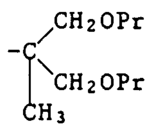 | blaustichig-rot |
| 63 | 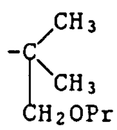 | blaustichig-rot |
| 64 | 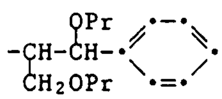 | blaustichig-rot |
| 65 | 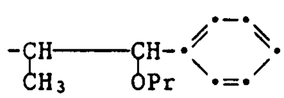 | blaustichig-rot |
| 66 | 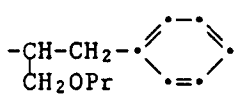 | blaustichig-rot |
| 67 | 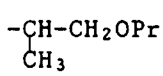 | blaustichig-rot |
| 68 | $-CH_2CH_2CH_2-OPr$ | blaustichig-rot |

Tabelle 4

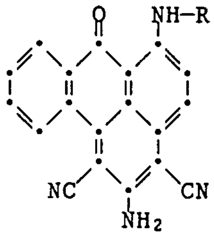

| Beispiel | R (Bz = 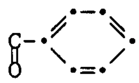 ) | Nuance auf Polyester |
|---|---|---|
| 69 | 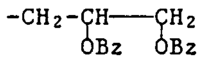 | blaustichig-rot |
| 70 | 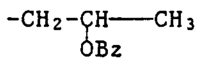 | blaustichig-rot |
| 71 | 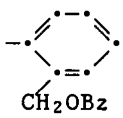 | rot-violett |
| 72 | 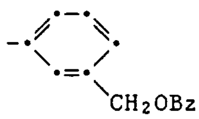 | rot-violett |
| 73 | 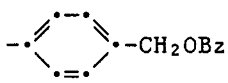 | rot-violett |
| 74 | 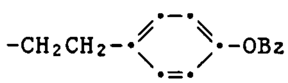 | blaustichig-rot |
| 75 | 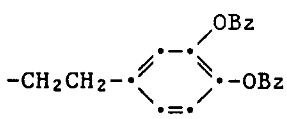 | blaustichig-rot |
| 76 | $-CH_2CH_2OBz$ | blaustichig-rot |
| 77 | 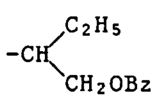 | blaustichig-rot |
| 78 | $-CH_2CH_2CH_2CH_2OBz$ | blaustichig-rot |
| 79 | 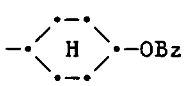 | blaustichig-rot |

Tabelle 4 (Fortsetzung)

| Beispiel | R (Bz = 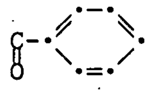 ) | Nuance auf Polyester |
|---|---|---|
| 80 | 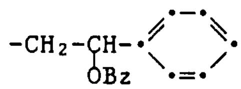 | blaustichig-rot |
| 81 | 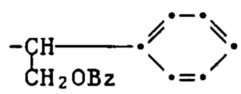 | blaustichig-rot |
| 82 | 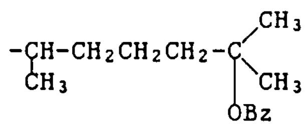 | blaustichig-rot |
| 83 | 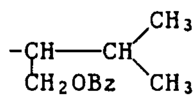 | blaustichig-rot |
| 84 | 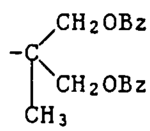 | blaustichig-rot |
| 85 | 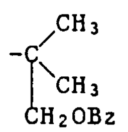 | blaustichig-rot |
| 86 | 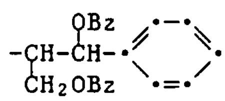 | blaustichig-rot |
| 87 | 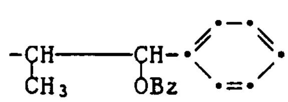 | blaustichig-rot |
| 88 | 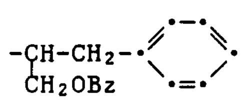 | blaustichig-rot |
| 89 | 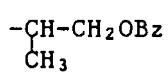 | blaustichig-rot |
| 90 | $-CH_2CH_2CH_2-OBz$ | blaustichig-rot |

Beispiel 91: Arbeitet man wie im Beispiel 1 beschrieben, setzt jedoch anstelle von 11,8 g 1-Hydroxyisobutylaminoanthrachinon eine äquivalente Menge 6-Chlor-1-hydroxyisobutylaminoanthrachinon ein, so erhält man bei ansonsten gleichem Arbeiten den Farbstoff der Formel

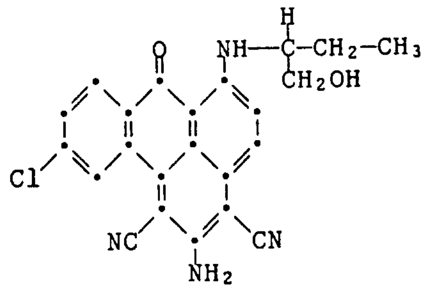

welcher Polyester in blaustichig roten Farbtönen mit guten Echtheiten färbt.

Beispiel 92: Man mischt 1 Teil des trockenen, coupagefreien Farbstoffs gemäss Beispiel 24 in einer Glasperlmühle zusammen mit 1 Teil Dinaphthylmethandisulfonat (Na-Salz) und Wasser und mahlt das Gemisch so lange, bis eine Korngrösse von etwa 2 µ oder kleiner erreicht ist. Die entstehende Paste, bestehend aus dem Farbstoff, Dispergator und Wasser, wird anschliessend mit 3 Teilen Natriumligninsulfonat versetzt. Die erhaltene Paste wird sodann der Sprühtrocknung unterworfen, wobei ein pulverförmiges Färbepräparat erhalten wird.

Dieses Färbepräparat kann zum Färben von Polyestermaterialien, z.B. nach dem HT-Verfahren, verwendet werden, wobei das Färbebad eine gute Dispersionsstabilität aufweist. Man erhält eine blaustichig-rote Polyesterfärbung mit guter Lichtechtheit.

Beispiel 93: 2 Teile des gemäss Beispiel 24 erhaltenen Farbstoffs werden in 4000 Teilen Wasser dispergiert. Zu dieser Dispersion gibt man 12 Teile des Natriumsalzes von o-Phenylphenol sowie 12 Teile Diammoniumphosphat und färbt 100 Teile Garn aus Polyethylenglykolterephthalat 90 Minuten lang bei 95 bis 98° in dieser Flotte.

Die Färbung wird anschliessend gespült und mit wässriger Natronlauge und einem Dispergator nachbehandelt. Man erhält so eine brillante licht- und sublimierechte blaustichig rote Färbung.

Beispiel 94: 1 Teil des gemäss Beispiel 24 erhaltenen Farbstoffs wird mit 2 Teilen einer 50%igen wässerigen Lösung des Natriumsalzes der Dinaphthylmethandisulfonsäure nass vermahlen und getrocknet.

Dieses Farbstoffpräparat wird mit 40 Teilen einer 10%igen wässrigen Lösung des Natriumsalzes der N-Benzylheptadecyl-benzimidazoldisulfonsäure verrührt und 4 Teile einer 40%igen Essigsäurelösung zugegeben. Durch Verdünnen mit Wasser wird daraus ein Färbebad von 4000 Teilen bereitet.

In dieses Bad geht man bei 50° mit 100 Teilen eines Polyesterfaserstoffes ein, steigert die Temperatur innert einer halben Stunde auf 120 bis 130° und färbt eine Stunde in geschlossenem Gefäss bei dieser Temperatur. Anschliessend wird gut gespült. Man erhält eine brillante blaustichig rote Färbung von guter Lichtechtheit.

Beispiel 95: Polyethylenglykolterephthalatgewebe wird auf einem Foulard bei 40° mit einer Flotte folgender Zusammensetzung imprägniert:

20 Teile des gemäss Beispiel 24 erhaltenen Farbstoffes fein dispergiert in
10 Teilen Natriumalginat,
20 Teilen Triethanolamin,
20 Teilen Octylphenolpolyglykoläther und
930 Teilen Wasser.

Das auf ca. 100 % abgequetschte Gewebe wird bei 100° getrocknet und anschliessend während 30 Sekunden bei einer Temperatur von 210° fixiert. Die gefärbte Ware wird mit Wasser gespült, geseift und getrocknet. Man erhält eine brillante, lichtechte blaustichig rote Färbung.

Beispiel 96: Ein zur Faserherstellung geeignetes unmattiertes Polyäthylenterephthalat-Granulat wird in einem verschliessbaren Gefäss zusammen mit 1 % Farbstoff gemäss Beispiel 24 auf einer Schüttelmaschine 15 Minuten geschüttelt. Die gleichmässig gefärbten Granulatkörner werden auf einer Schmelzspinnanlage (285°C ± 3°, Verweilzeit in der Spinnmaschine ca. 5 Minuten) zu Fäden versponnen, die auf einer Streckzwirnanlage verstreckt und aufgespult werden. Man erhält eine brillante blaustichig rote Färbung, die sich durch hervorragende Lichtechtheit und Sublimationsechtheit auszeichnet.

**Patentansprüche**

**1.** Verbindungen der Formel

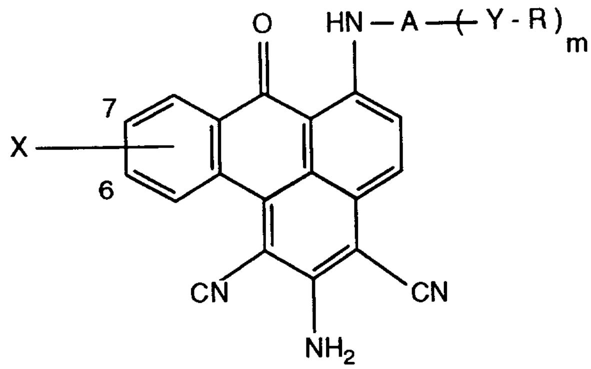

(1),

worin
X Wasserstoff, Halogen, Nitro oder $NH_2$ ist,
R Wasserstoff oder einen Rest $-COR^1$ bedeutet, worin $R^1$ eine gegebenenfalls substituierte Alkyl- oder Arylgruppe darstellt,
Y O oder S ist,
m 1, 2 oder 3 ist und
A einen aliphatischen, aromatischen oder araliphatischen Rest bedeutet, wobei falls R Wasserstoff, Y O, X Wasserstoff oder ein in der Position 6 oder 7 gebundenes Halogen und A ein aliphatischer Rest sind, darf A nur $C_2$- $C_5$-Alkylen bedeuten.

**2.** Verbindungen gemäss Anspruch 1, worin X Wasserstoff bedeutet.

**3.** Verbindungen gemäss einem der Ansprüche 1 oder 2, worin Y -O-bedeutet.

**4.** Verbindungen gemäss einem der Ansprüche 1-3, worin R einen Rest

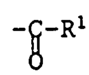

bedeutet.

**5.** Verbindungen gemäss einem der Ansprüche 1-4, worin $R^1$ $C_1$-$C_6$-Alkyl, welches unsubstituiert ist oder durch Phenyl oder Phenoxy substituiert ist, wobei die Phenylgruppe in den beiden letztgenannten Resten durch $C_1$-$C_4$-Alkyl, $C_1$-$C_4$-Alkoxy oder Phenoxy substituiert sein kann, oder Phenyl, welches unsubstituiert ist oder durch $C_1$-$C_4$-Alkyl substituiert ist, bedeutet.

**6.** Verbindungen gemäss Anspruch 5, worin $R^1$ $C_1$-$C_3$-Alkyl oder Phenyl bedeutet.

**7.** Verbindungen gemäss einem der Ansprüche 1-6, worin m 1 oder 2, vor allem 1 bedeutet.

**8.** Verbindungen gemäss einem der Ansprüche 1-7, worin A einen $C_2$-$C_5$-Alkylenrest oder einen Phenylen-$C_2$-$C_5$-alkylenrest bedeutet.

**9.** Verbindungen der Formel

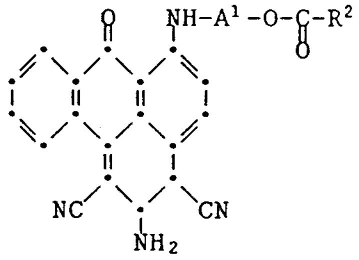

(2)

worin
$A^1$ einen $C_2$-$C_5$-Alkylenrest oder einen Phenylen-$C_2$-$C_5$-Alkylenrest und
$R^2$ $C_1$-$C_3$-Alkyl oder Phenyl bedeutet.

**10.** Verfahren zur Herstellung der Verbindungen der Formel (1) gemäss Anspruch 1, dadurch gekennzeichnhet, dass man ein substituiertes Aminoanthrachinon der Formel

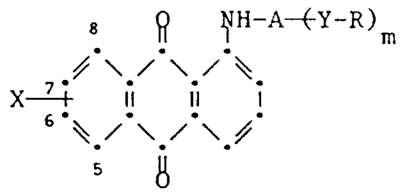

(3)

worin X, Y, A, m und R die unter der Formel (1) angegebene Bedeutung aufweisen, in einem inerten Lösungsmittel in Gegenwart von Titantetrachlorid und einem tert.Amin mit Malodinitril umsetzt.

**11.** Verwendung der Verbindungen der Formel (1) gemäss Anspruch 1 zum Färben oder Bedrucken von halbsynthetischen oder synthetischen hydrophoben Fasermaterialien, insbesondere Textilmaterialien aus linearen aromatischen Polyestern.

**12.** Das gemäss Anspruch 11 gefärbte oder bedruckte hydrophobe Material.

**Claims**

**1.** A compound of the formula

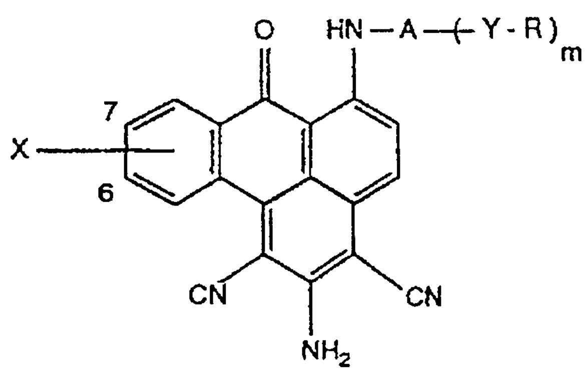

(1),

in which X is hydrogen, halogen, nitro or $NH_2$, R is hydrogen or a radical -$COR^1$ in which $R^1$ is a substituted or unsubstituted alkyl or aryl group, Y is O or S, m is 1, 2 or 3, and A is an aliphatic, aromatic or araliphatic radical; if R is hydrogen, Y is O, X is hydrogen or is halogen attached in position

6 or 7 and A is an aliphatic radical, A can only be $C_2$-$C_5$alkylene.

2. A compound according to claim 1 in which X is hydrogen.

3. A compound according to either of claims 1 and 2 in which Y is -O-.

4. A compound according to any one of claims 1-3 in which R is a radical

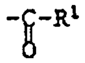

.

5. A compound according to any one of claims 1-4 in which $R^1$ is $C_1$-$C_6$alkyl, which is unsubstituted or substituted by phenyl or phenoxy, where the phenyl group in the two last-mentioned radicals can be substituted by $C_1$-$C_4$alkyl, $C_1$-$C_4$alkoxy or phenoxy, or is phenyl, which is unsubstituted or substituted by $C_1$-$C_4$alkyl.

6. A compound according to claim 5 in which $R^1$ is $C_1$-$C_3$alkyl or phenyl.

7. A compound according to any one of claims 1-6 in which m is 1 or 2, in particular 1.

8. A compound according to any one of claims 1-7 in which A is a $C_2$-$C_5$alkylene radical or a phenylene-$C_2$-$C_5$alkylene radical.

9. A compound of the formula

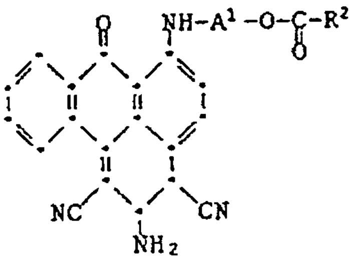

(2)

in which $A^1$ is a $C_2$-$C_5$alkylene radical or a phenylene-$C_2$-$C_5$alkylene radical and $R^2$ is $C_1$-$C_3$alkyl or phenyl.

10. A process for the preparation of the compounds of the formula (1) according to claim 1, which comprises reacting a substituted aminoanthraquinone of the formula

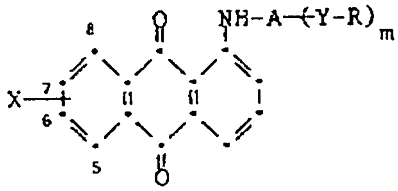

(3)

in which X, Y, A, m and R are as defined in formula (1) with malodinitrile in an inert solvent in the presence of titanium tetrachloride and a tertiary amine.

11. Use of the compounds of the formula (1) according to claim 1 for the dyeing or printing of semisynthetic or synthetic hydrophobic fibre materials, in particular textile materials made of linear aromatic polyesters.

12. The hydrophobic material dyed or printed according to claim 11.

**Revendications**

1. Composés de formule

(1)

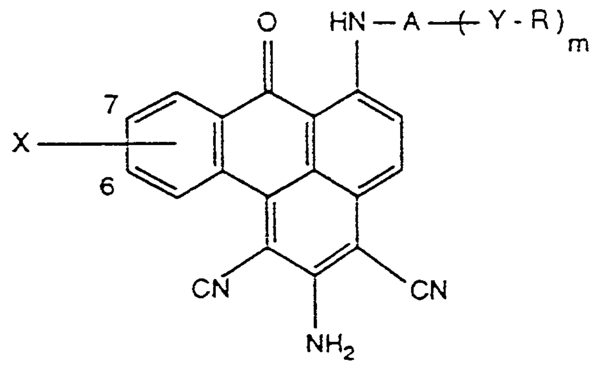

dans laquelle
X représente un atome d'hydrogène ou d'halogène, un groupe nitro ou $NH_2$,
R représente un atome d'hydrogène ou un résidu -CO-$R^1$, dans lequel $R^1$ représente un groupe alkyle ou aryle pouvant être substitué,
Y représente un atome d'oxygène ou de soufre,
m est égal à 1, 2 ou 3 et
A représente un résidu aliphatique, aromatique ou araliphatique,
avec la réserve que, lorsque R est un atome d'hydrogène, Y un atome d'oxygène, X un atome d'hydrogène ou un atome d'halogène en position 6 ou 7 et A un résidu aliphatique, alors A ne peut être qu'un résidu alkylène en $C_2$-$C_5$.

2. Composés conformes à la revendication 1 dans lesquels X représente un atome d'hydrogène.

3. Composés conformes à une des revendications 1 ou 2 dans lesquels Y représente un groupe -O-.

4. Composés conformes à une des revendications 1 à 3 dans lesquels R représente un résidu -CO-$R^1$.

5. Composés conformes à une des revendications 1 à 4 dans lesquels $R^1$ représente un groupe alkyle en $C_1$-$C_6$, substitué ou non parun groupe phényle ou phénoxy, les groupes phényle de ces deux derniers groupes pouvant être substitués par des résidus alkyle en $C_1$-$C_4$, alcoxy en $C_1$-$C_4$ ou phénoxy, ou un groupe phényle substitué ou non par un groupe alkyle en $C_1$-$C_4$.

6. Composés conformes à la revendication 5 dans lesquels $R^1$ représente un résidu alkyle en $C_1$-$C_3$ ou phényle.

7. Composés conformes à une des revendications 1 à 6 dans lesquels m est égal à 1 ou 2, surtout égal à 1.

8. Composés conformes à une des revendications 1 à 7 dans lesquels A représente un résidu alkylène en $C_2$-$C_5$ ou un résidu phénylène-alkylène-(en $C_2$-$C_5$).

**9.** Composés de formules

(2)

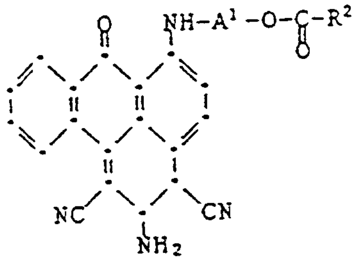

dans laquelle
$A^1$ représente un résidu alkylène en $C_2$-$C_3$ ou phénylène-alkylène-(en $C_2$-$C_5$), et
$R^2$ représente un résidu alkyle en $C_1$-$C_3$ ou phényle.

**10.** Procédé pour la préparation de composés de formule (1) conformes à la revendication 1, caractérisé en ce que l'on fait réagir une aminoanthraquinone substituée de formule

(3)

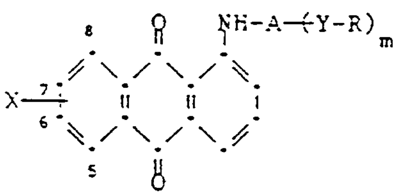

dans laquelle X, Y, A, m et R ont la signification indiquée dans la formule (1), avec du malodinitrile dans un solvant inerte en présence de tétrachlorure de titane et d'une amine tertiaire.

**11.** Utilisation des composés de formule (1) conformes à la revendication 1 pour la teinture ou l'impression de matériaux fibreux hydrophobes syntnétiques ou semi-synthétiques, en particulier de matériaux textiles en polyesters aromatiques linéaires.

**12.** Matériau hydrophobe teint ou imprimé conformément à la revendication 11.